# FASCICULE DE BREVET EUROPEEN

(11) **EP 3 340 927 B1**
(45) Date de publication et mention de la délivrance du brevet: **09.10.2024**
(21) Numéro de dépôt: 16763464.1
(22) Date de dépôt: 26.08.2016
(51) Int. Cl.: A61F 2/08, A61B 17/04, A61B 17/06

(54) **SYSTEME DE FIXATION INTRA-OSSEUSE D'UN FILIN SOUPLE DESTINE A L'ANCRAGE OSSEUX DE TISSUS LIGAMENTAIRES**
SYSTEM ZUR INTRAOSSÄREN BEFESTIGUNG EINES FLEXIBLEN DRAHTS ZUR VERANKERUNG VON BÄNDERGEWEBE AN KNOCHEN
SYSTEM FOR THE INTRAOSSEOUS ATTACHMENT OF A FLEXIBLE WIRE INTENDED FOR ANCHORING LIGAMENT TISSUE TO BONE

(30) Priorité: 26.08.2015 BE 201505539; 18.01.2016 WO PCT/EP2016/050929
(43) Date de publication de la demande: 04.07.2018
(73) Titulaire: Medacta International SA, 6874 Castel San Pietro (CH)
(72) Inventeur: COLLETTE, Michel, 1180 Bruxelles (BE)
(74) Mandataire: Inchingalo, Simona
(86) Numéro de dépôt international: PCT/EP2016/070265
(87) Numéro de publication internationale: WO 2017/032901

(56) Documents cités:
- WO-A1-2004/045465
- WO-A1-2007/147634
- WO-A2-2007/070024
- US-A- 4 950 270

## Description

### Arrière-plan de l'invention

L'ancrage osseux de tissus mous de type capsulaire ou ligamentaire constitue un des impératifs chirurgicaux les plus fréquents après traumatisme sportif. La fixation d'une greffe ligamentaire dans le tissu osseux en vue de reconstruire le ligament croisé antérieur rompu est un exemple parmi les plus fréquents (mais non exclusif) de ce type de procédé chirurgical. Cette application a été choisie pour exposer la présente invention mais il va de soi que celle-ci peut s'appliquer également à d'autres structures anatomiques.

La reconstruction chirurgicale du ligament croisé antérieur (LCA) rompu s'effectue aujourd'hui le plus souvent au moyen d'une greffe tendineuse prélevée aux dépens soit du tendon rotulien soit des tendons des muscles ischio-jambiers postéro internes de la cuisse et du genou, le droit interne et le demi tendineux (DIDT).

Bien que les greffes de tendon rotulien soient réputées plus efficaces sur le plan mécanique, les greffes de DIDT sont de plus en plus utilisées car les effets secondaires indésirables liés à leur prélèvement sont en général moins importants qu'avec les greffes de tendon rotulien.

Classiquement, les greffes de DIDT se construisent en repliant sur eux-mêmes en leur milieu chacun des deux tendons prélevés (droit interne et demi tendineux) pour obtenir une greffe à quatre brins tendineux mesurant de 12 à 15 cm environ. La longueur du ligament croisé à reconstruire n'étant que de 3 cm environ, on se rend compte que 70 à 80 % de la substance tendineuse prélevée sert uniquement à réaliser la fixation de la longueur utile de la greffe (3cm intra-articulaire).

Le demandeur a décrit précédemment un nouveau système qui a fait l'objet de demandes de brevet (voir publication PCT WO20041045465 et WO2007/147634), permettant de réaliser la même intervention en prélevant un seul tendon, le demi tendineux et en le repliant deux fois sur lui-même pour obtenir, comme dans les techniques classiques, une greffe à quatre brins tendineux mais avec la conséquence inévitable d'obtenir une greffe de dimension réduite (5 ou 6 cm environ). L'économie de tissu tendineux ainsi réalisée constitue incontestablement un progrès par rapport aux techniques classiques mais elle induit de nouvelles difficultés techniques relatives à la fixation osseuse de cette greffe courte. En effet, la quantité réduite de tissus ligamentaire introduit dans l'os ne permet plus d'utiliser les procédés de fixation classiques telle que la compression de la greffe dans le tunnel osseux par une vis de serrage introduite entre la greffe et la paroi intérieure du tunnel osseux (vis d'interférence) ou encore la suspension de la boucle ligamentaire par un organe axiforme solide transfixiant la greffe et fixé dans l'os avoisinant le tunnel osseux, perpendiculairement à l'axe de la greffe.

La pose d'une greffe courte impose donc l'utilisation de relais synthétiques prolongeant la greffe à ses deux extrémités et la reliant à son organe de fixation.

Un premier procédé consiste à ancrer l'organe de fixation au niveau de l'os cortical, c'est-à-dire à la sortie du tunnel osseux dans lequel se trouve la greffe. L'os cortical est sans nul doute la zone osseuse la plus résistante offrant donc au système d'ancrage une capacité de résistance à la traction extrêmement élevée (plus de 1000 Newtons). Cependant, plus courte sera la greffe, plus long devra être le relais synthétique avant d'atteindre le niveau cortical où se situe l'organe de fixation. Or, il a été clairement et maintes fois démontré expérimentalement, d'une part que l'élasticité du montage augmente proportionnellement à la longueur du relais synthétique et d'autre part que cette élasticité affecte négativement les propriétés mécaniques du système. En effet, l'élasticité des relais synthétiques favorise les micros mouvements axiaux ou transversaux à l'intérieur du tunnel osseux ce qui entraîne inévitablement un relâchement de la tension de la greffe et progressivement un élargissement des tunnels osseux qui l'abritent.

Un deuxième procédé consiste à ancrer l'organe de fixation à proximité de la greffe en réduisant ainsi au maximum la longueur du relais synthétique entre la greffe et l'organe de fixation et donc par conséquent l'élasticité globale du montage. Ce système décrit la suspension de la greffe courte par deux bandelettes textiles (polyéthylène téréphtalate) fixées à l'intérieur même du tunnel osseux par une vis spéciale appelée vis TLS (Tape Locking Screw) introduite de dehors en dedans, en direction de la greffe et pratiquement jusqu'à son contact ce qui neutralise effectivement l'élasticité du relais textile.

Bien que la technique TLS connaisse aujourd'hui un essor considérable dans le monde, (plus de 30 000 cas opérés à ce jour), elle comporte des inconvénients résiduels qui ont été en partie décrits dans le document de demande de brevet WO2013/139985 intitulé « Système de fixation d'une greffe ligamentaire ».

L'expérience clinique a en effet permis d'identifier plusieurs facteurs susceptibles d'affecter la qualité du résultat final :
- la qualité de l'os dans lequel s'effectue l'ancrage. Plus l'os est tendre, moins la fixation sera solide.
- La qualité du vissage : l'excès ou le manque de pénétration de la vis dans le tunnel osseux est susceptible d'affecter la solidité du montage. Il en va de même si l'axe de pénétration de la vis diverge par rapport à l'axe du tunnel osseux dans lequel passent les bandelettes .
- La qualité de déploiement du ruban au sein du tunnel osseux demeure un facteur incontrôlable à maîtriser, susceptible également d'affecter la performance du vissage du ruban.
- Pour permettre le passage des bandelettes dans les tunnels osseux, le diamètre de creusement de ces tunnels doit être au moins de 4,5 mm. Bien que cela constitue déjà un progrès par rapport aux techniques classiques imposant de creuser les tunnels au diamètre de la greffe (8 à 10 mm), cet impératif de diamètre de creusement affecte quelque peu le caractère mini invasif recherché par la technique.
- Après creusement du tunnel, la technique TLS impose de tarauder l'entrée du tunnel osseux pour préparer le logement de la vis. Bien que relativement simple de réalisation, ce geste constitue néanmoins une étape supplémentaire par rapport à d'autres techniques ou ce geste technique n'existe pas, prolongeant donc ainsi le temps opératoire qui idéalement doit être réduit au minimum possible.
- Enfin, le volume relativement important de la vis TLS compromet quelque peu l'économie de tissus osseux à laquelle vise la méthode et par conséquent réduit son caractère mini invasif. Cet inconvénient paraît particulièrement marquant en cas de reconstruction du ligament croisé antérieur chez l'enfant ou les structures osseuses sont de petite dimension.

Le document de brevet WO2013/139985 (système TLS 3) proposait donc un système visant à corriger plusieurs faiblesses du sytème TLS:
- Le remplacement des rubans par un filin de suspension permet de réduire le diamètre des tunnels osseux nécessaires à son passage dans l'os.
- L'utilisation d'une cheville de serrage du filin en son centre permet de s'affranchir des problèmes liés à la qualité de l'os environnant, à la qualité du vissage et aux problèmes liés au déploiement du ruban au sein du tunnel osseux.

Cependant, comme dans le système TLS initial, le logement de la cheville de serrage nécessite de créer une logette osseuse spécifique ce qui alourdit la réalisation technique de l'intervention et affecte négativement le caractère mini invasif de la procédure alors qu'il s'agit d'un des objectifs principaux de cette nouvelle technique.

La présente invention conserve l'utilisation d'un filin de suspension (dont les avantages par rapport aux bandelettes de suspension ont été décrits plus haut), conserve la possibilité de neutraliser l'élasticité du filin comme dans la technique TLS originale en introduisant l'organe de fixation dans le tunnel osseux pratiquement jusqu'au contact de la greffe, mais supprime la nécessité de créer une logette osseuse spécifique destinée à accueillir l'organe de fixation.

La texture et la dimension des bandelettes de suspension utilisées dans la technique TLS originale permettaient d'obtenir une fixation solide par coincement du ruban entre la vis de serrage et la paroi du tunnel osseux.

Le même résultat ne peut malheureusement pas être obtenu par vissage d'un simple filin dont la surface lisse et très réduite exposerait au risque de glissement pour une contrainte en traction nettement inférieure à celle requise pour la fixation d'un ligament (600 à 900 Newtons). Par contre, en fixant sur le filin plusieurs petits nodules solides, on obtient un filin souple corpusculaire, devenu propice à la fixation intra-osseuse par interférence avec le relief d'une simple vis introduite dans le même tunnel osseux.

### Discussion comparative complémentaire de l'art antérieur le plus proche

La recherche de l'art antérieur a permis de révéler l'existence d'une demande de brevet WO 2012/058301 décrivant déjà l'utilisation d'un filin souple muni de multiples corpuscules, destiné notamment à la fixation sur l'os de tissu ligamentaire rompu. Le mécanisme de blocage et l'effet mécanique obtenu sont cependant entièrement différents.

En effet, dans le système de la demande WO 2012/058301 , le filament se déplace en direction de l'organe de blocage. Cela signifie que le positionnement du filin corpusculaire et son blocage dans le tunnel osseux se produisent de façon simultannée : dès que le filin se déplace dans le tunnel, il se bloque automatiquement dans sa nouvelle position , sans possibilité de revenir en arrière. En pratique, si le chirurgien se rend compte que la greffe est trop tendue, le système de blocage tel que décrit dans cette invention, ne lui permet plus de détendre le filin de suspension , puisque par définition, le filin se bloque dans un seul sens de déplacement.

Avec le dispositif de la présente invention, comme on le comprendra plus amplement dans la description qui suit, le positionnement du filin dans le tunnel d'une part et le processus de blocage du filin d'autre part, constituent deux étapes strictement indépendantes l'une de l'autre. En pratique, le chirurgien commence par positionner librement sa greffe dans le tunnel osseux en agissant par traction sur le filin, s'il veut tendre la greffe, ou par traction sur la greffe s'il veut la détendre, et ce n'est qu'après avoir réglé le positionnement et la tension de la greffe qu'il va introduire la vis de blocage qui se déplace dans le tunnel, le long du filin qui, lui-même, reste parfaitement immobile. Si même après l'introduction de la vis, le chirurgien devait constater une erreur de réglage dans la tension, selon la présente invention, le dispositif permet encore de dévisser l'organe de fixation, reprendre le réglage de la tension et réintroduire la vis de blocage.

En outre, la longueur nécessaire du filin de suspension correspond à la distance entre l'extrémité du ligament à fixer et la zone d'appui de l'organe de blocage. Dans l'exemple de notre greffe courte de ligament croisé, l'extrémité de la greffe se trouve à proximité de l'orifice d'entrée intra-articulaire du tunnel osseux alors que l'organe de blocage devrait se situer au niveau cortical, c'est à dire à la sortie de ce même tunnel, tels que l'illustre d'ailleurs la figure 12 du document descriptif original. Ce procédé produirait donc un système de suspension mécaniquement défavorable comme nous l'avons déjà expliqué plus haut, comprenant un long relai souple, par conséquent élastique, entre la greffe, siégeant à l'entrée intra-articulaire du tunnel osseux, et l'organe de blocage, siégeant lui au niveau cortical, c'est à dire à la sortie du tunnel osseux.

Dans le présent dispositif ou système au contraire, la vis de blocage progresse de dehors en dedans, en direction de la greffe et vient bloquer le filin pratiquement jusqu'au contact de la greffe, neutralisant ainsi l'élasticité potentielle du système de suspension.

Enfin, puisque l'ancrage est assuré par le blocage de deux corpuscules à travers les deux orifices anti retour, la résistance à l'arrachement de l'organe tissulaire ainsi fixé se limite à la résistance à l'arrachement cumulée de deux corpuscules sur le filin.

Dans le système de la présente invention, le blocage des corpuscules, et donc du filin, se produit à l'intérieur même du tunnel osseux par l'introduction dans ce tunnel d'une vis dont le relief interagit avec le relief de tous les corpuscules. Plusieurs types d'interaction différents peuvent être envisagés, dépendant essentiellement du mode de fabrication du filin corpusculaire et du type de vis utilisée.

Une autre demande de brevet WO 2007/070024 décrit également un système de filin corpusculaire permettant d'obtenir un ancrage par vis.

Ce document de brevet décrit certes l'usage d'un filament corpusculaire mais qui n'agit que s'il est introduit en grand nombre dans une cavité osseuse dont on cherche à provoquer l'expansion par bourrage. La compaction des innombrables corpuscules reliés entre eux par des filaments, recrée ainsi un environnement dense et suffisamment compact que pour y créer, si nécessaire un ancrage par vis. Cette invention décrit donc un moyen permettant d'ancrer une vis dans une cavité osseuse préalablement bourrée de filins corpusculaires multiples.

A l'inverse, notre invention décrit un dispositif visant à ancrer non pas une vis mais un seul filin corpusculaire dans un fin tunnel osseux, grâce à une vis.

US4950270 divulgue un dispositif de fixation de tissu mou à un tissu osseux comprenant: une vis canulée et une broche de guidage apte à coopérer avec ce vis. U

Les avantages du présent système par rapport au système de l'art antérieur sont multiples :
- l'ajustement du positionnement de la greffe et sa mise en tension s'effectuent de façon totalement libre et indépendante du processus de blocage qui ne survient que secondairement, pour «fixer» la position choisie par le chirurgien. En cas de problème, la vis peut d'ailleurs être ôtée, puis réintroduite après avoir réajusté la tension selon le souhait du chirurgien.
- la vis bloque tous les corpuscules fixés sur le filin et neutralise ainsi son élasticité pratiquement jusqu'au contact de la greffe (comme dans le système TLS original).
- la vis peut être introduite directement dans le tunnel, sans préparation d'un logement particulier (comme l'exige le système TLS).
- les corpuscules étant fixés en série sur le filin, la résistance à l'arrachement de la greffe correspond à la résistance à l'arrachement cumulée de tous les corpuscules enclavés par la vis. Si par exemple la résistance à l'arrachement d'un corpuscule sur le filin est de 10 kg, la résistance d'un filin comprenant 10 corpuscules enclavés sera d'environ 100 kg.
- tout en conservant des propriétés mécaniques très performantes, la mise en oeuvre du système s'effectue par une technique particulièrement mini-invasive (tunnels, filin et vis de calibre extrêmement faible)
- Bien que dans le dispositif et le processus technique de l'invention la vis est introduite de dehors en dedans, le faible calibre de cette vis peut permettre, si on le souhaite, de l'introduire de dedans en dehors, comme dans les techniques de ligamentoplasties classiques in-out.

### RESUME DE L'INVENTION

La présente invention propose un système permettant de suspendre et de fixer une greffe ligamentaire à travers des tunnels osseux réduits à leur plus petite dimension (2-3 mm) et sans devoir creuser une logette osseuse spécifique destinée à accueillir l'organe de fixation.

Ce système se compose d'un filin très solide sur lequel sont fixés plusieurs corpuscules solides, et d'un organe de blocage constitué par une vis introduite dans le même tunnel osseux parallèlement au filin corpusculaire. Le filin de suspension peut se composer d'un seul filament se terminant à une de ses extrémités par une anse fermée destinée à suspendre la greffe. Le filin de suspension peut également se terminer par une aiguille sertie permettant d'effectuer un laçage de l'extrémité du ligament à refixer sur l'os. Il peut également être replié sur lui-même pour former une anse naturelle destinée à suspendre la greffe, chaque brin de suspension pouvant être garni de corpuscules. Le filin porteur de la greffe est apte à être attiré dans un tunnel osseux dont le diamètre est à peine supérieur au diamètre du filin qui, selon l'application pourrait varier entre 0,5 et 2mm. Le verrouillage du filin (et donc de la greffe sous-jacente) est obtenue par l'introduction dans le même canal osseux d'une vis à os classique,cheminant dans le tunnel osseux parallèlement au filin. Si la technique chirurgicale le demande (ex : ligamentoplastie) , on pourra utiliser une vis perforée en son centre selon son axe longitudinal, d'usage courant en othopédie, de manière à pouvoir l'enfiler sur une fine broche guide , préalablement disposée dans le tunnel osseux et destinée à guider avec certitude le trajet de la vis au sein du tunnel. Le mode de réalisation classique consiste à introduire la vis de dehors en dedans, en direction de la greffe mais, en fonction des circonstances et de l'objectif à atteindre, rien ne s'oppose à introduire la vis à travers le tissu ligamentaire lui-même et la faire progresser de dedans en dehors, en s'éloignant de la greffe, à la manière des techniques de ligamentoplastie classiques in-out. Selon le type de corpuscules utilisés, le blocage est obtenu soit par refoulement et encastrement des corpuscules dans la paroi osseuse, soit par emprisonnement des corpuscules dans l'espace situé entre deux filets adjacents, entre l'âme de la vis et l'os avoisinant, soit par un mécanisme combinant les deux effets .

Dans **un premier mode de réalisation,** les corpuscules peuvent être oblongs et de dimension longitudinale supérieure à la distance du pas de vis. Dans ce cas, l'introduction de la vis dans le tunnel provoque l'expulsion du filament corpusculaire à l'extérieur du corps de vis, les corpuscules venant véritablement s'encastrer dans le tissu osseux avoisinant. La forme du corpuscule peut d'ailleurs être optimisée pour favoriser cette pénétration intra-osseuse.

Dans un **second mode de réalisation,** la distance entre les corpuscules ainsi que leur taille, sont ajustées pour qu'ils viennent se loger dans l'espace situé entre les filets de la vis, lesquels créant ainsi un obstacle pratiquement infranchissable au glissement des corpuscules . En effet, ils sont ainsi emprisonnés, audessus et en dessous d'eux par le filet de la vis et latéralement, par la paroi rigide du tunnel osseux.

Les deux modes peuvent avantageusement se combiner par l'utilisation de corpuscules dont les dimensions permettent à la fois l'enclavement entre les filets de la vis (par l'ajustement de la distance intercorpusculaire) et la protrusion dans le tissu osseux (par l'ajustement de leur forme et de leur diamètre) (voir plus loin : descriptif des corpuscules).

Quel que soit le mode de réalisation utilisé, il existe des exigences de forme et de dimension du couple vis-corpuscule qui constituent une condition essentielle au bon fonctionnement du système décrit.

Il est par exemple important que la vis ait une forme conique , principalement au voisinage de sa pointe qui doit être aussi fine que possible. Il faut en effet qu'en progressant, dans le tunnel, la vis laisse au corpuscule suffisamment d'espace pour pouvoir s'insinuer entre sa pointe et la paroi du tunnel osseux, afin de s'y encastrer. On comprend aisément qu'une vis trop massive dont l'extrémité occuperait tout le diamètre du tunnel osseux provoquerait le refoulement hors du tunnel des corpuscules rencontrés, empêchant ainsi toute possibilité d'ancrage du filin dans le tunnel.

Pour être efficace, l'interaction du couple vis-corpuscule nécessite donc un ajustement précis de la forme et de la taille de la vis par rapport à la forme et la taille des corpuscules, mais aussi un ajustement très précis de la taille de ces composants par rapport au diamètre du tunnel osseux, c'est à dire aux dimensions de la tarrière de creusement des tunnels.

### DESCRIPTION DETAILLEE DE L'INVENTION

### 1. Le filin

Pour répondre aux exigences de la présente invention, le filin de suspension doit être à la fois très résistant aux efforts de traction, aussi rigide que possible et de taille aussi faible que possible pour permettre son insertion à travers des tunnels osseux de calibre minimum.

Parmi les différents matériaux textiles bio compatibles disponibles, la fibre Dyneema^{®} possède des caractéristiques mécaniques répondant particulièrement bien aux exigences du système proposé. Elle est largement utilisée dans divers domaines exigeant des cordages très résistants (sports nautiques, parachutisme, alpinisme etc.) mais elle fait également partie de diverses applications médicales. Il s'agit d'une fibre de polyéthylène de haute densité extrêmement résistante à la traction (15 fois plus résistantes que le fil d'acier) et de rigidité élevée (faible élasticité) ce qui correspond exactement aux caractéristiques du système de suspension que nous recherchons. A titre d'exemple, un filin de 1,5 à 2 mm peut résister à des charges de 200 à 300 kg ce qui constitue une limite de résistance deux à trois fois supérieures à celle nécessaire dans le cadre de l'invention. Cela signifie que grâce à ce type de fibres, il est possible de fabriquer un filin de très faible diamètre qui, après ajout des corpuscules atteindra un diamètre final de 1,5 à 2 mm pouvant être introduit dans un tunnel de 3 à 4 mm ce qui constitue un progrès significatif par rapport au système TLS qui impose un creusement osseux de 4,5 mm et à fortiori par rapport aux techniques classiques qui, comme déjà mentionné, imposent de creuser les tunnels osseux au diamètre de la greffe (8 à 10 mm).

Le filin selon l'invention se compose donc d'un filament, extrêmement résistant de très faible diamètre, sur lequel seront fixés un ou plusieurs corpuscules destinés à neutraliser toute possibilité de glissement après la mise en place de l'organe de blocage (vis). Différents modes de réalisation permettent de répondre aux variations de circonstances et d'objectifs à atteindre.

Par exemple, le filin peut se composer d'un seul filament dont une extrémité est repliée et refixée a lui-même (par épissures, suture, sertissage etc.) en formant une anse libre destinée à suspendre une boucle ligamentaire. Ou encore, l'extrémité du filin peut se terminer par une aiguille sertie permettant de faufiler l'extrémité du ligament à refixer. Ou encore, le filament peut être replié sur lui-même en créant ainsi une anse capable de cravater et suspendre une boucle ligamentaire, chaque section du filin située de part et d'autre de l'anse pouvant alors être garnie de corpuscules. Certaines applications pourraient nécessiter l'usage d'un simple filin corpusculaire sans formation d'une anse à son extrémité.

### 2. Les corpuscules

Ce sont des petits organes solides fixés ou intégrés au filin de manière à créer le long dudit filin un relief anti-glissement destiné à interagir avec le relief de l'organe de blocage (vis). Ces corpuscules seront fabriqués préférentiellement au moyen d'une matière composite biocompatible (ex: Peek, PLLA etc..) Mais tout autre matériau solide et biocompatible pourrait être utilisé (ex : métal etc.).

La fixation du corpuscule sur le filin doit être aussi solide que possible car c'est la résistance à l'arrachement de chaque corpuscule multipliée par le nombre de corpuscules qui déterminera la résistance globale à l'arrachement du filin lui-même et donc de la greffe ligamentaire qu'il suspend.

Le procédé de fixation des corpuscules sur le filin dépend étroitement du type de matériau choisi. N'importe quel procédé de fabrication (chimique, physique, mécanique etc.) peut être utilisé à condition qu'il permette d'obtenir une fixation solide sur le filin.

Les caractéristiques de forme et de dimensions des corpuscules jouent un rôle essentiel sur le type de mécanisme de blocage qui se produit sous l'effet de l'introduction de la vis.

La création de noeuds dans le filin lui-même, disposés à distance régulière en fonction des dimensions de la vis constitue un mode particulier de réalisation des corpuscules.

Plusieurs types de mécanismes de blocage sont envisageables selon la taille et la forme des corpuscules :
*a. Le corpuscule oblong dont la dimension longitudinale dépasse la distance séparant 2 filets adjacents de la vis.*
   Sa forme peut être cylindrique mais sera plus volontiers losangique ou trapézoïdale de façon à favoriser la pénétration des arrêtes au sein du tissu osseux
   Dans ce cas de figure, en pénétrant dans le tunnel osseux, la vis aura pour effet de repousser les corpuscules en dehors de l'axe du tunnel osseux, provoquant d'une part, l'encastrement de chaque corpuscule au sein du tissu osseux avoisinant, et d'autre part, l'empreinte du filet de la vis dans la paroi du corpuscule, les deux effets neutralisant le glissement au niveau de l'interface corpuscule - os.
*b. Le corpuscule court dont le diamètre est ajusté pour permettre son enclavement entre 2 filets adjacents de la vis.*

Sa forme est généralement sphérique mais toute autre forme comportant des arrêtes favorisant sa pénétration osseuse sera préférée si la dimension des corpuscules le permet.

Dans ce cas de figure, la vis aura pour effet d'emprisonner le corpuscule dans la concavité située entre les filets de la vis, le corpuscule se trouvant ainsi coincé d'une part entre la paroi du filet sus et sous-jacent et d'autre part entre l'âme de la vis et l'os avoisinant. Ce mécanisme requiert évidemment à la fabrication un ajustement précis entre les dimensions de la vis et la longueur du filament entre chaque corpuscule.

### 3. La vis

Selon l'invention, après avoir introduit le filin dans le tunnel osseux et ajusté sa tension selon le souhait du chirurgien, il suffit d'introduire dans ce même tunnel une vis dont le relief interagit avec les corpuscules du filin et neutralise complètement sa capacité de glissement.

Dans l'exemple de la ligamentoplastie, Il est essentiel pour obtenir l'effet de blocage, que la vis chemine dans le tunnel osseux selon un trajet rigoureusement parallèle à l'axe du filin. Cette exigence est rencontrée par l'utilisation d'une vis cannulée, d'utilisation courante en chirurgie othopédique. Il s'agit d'une vis creuse selon son axe longitudinal, pouvant coulisser sur une fine broche guide introduite au préalable dans le tunnel osseux, parallèlement au filin corpusculaire. L'utilisation d'une telle broche guide permet en outre de réaliser le placement de la vis à travers la peau et les tissus sous-cutanés, sans devoir aborder chirurgicalement la surface osseuse où se situe la sortie du tunnel osseux. Rappelons que la vis peut produire son effet de coincement
- soit par refoulement simple des corpuscules au sein de la paroi du tunnel osseux (corpuscules oblongs). Dans ce cas, la distance séparant les filets de la vis sera inférieure à la longueur de l'axe longitudinal des corpuscules.
- soit par emprisonnement du corpuscule dans l'espace circonscrit par la paroi de deux filets adjacents, l'âme de la vis et l'os avoisinant. Cette configuration requiert cependant quelques spécifications très précises à la fabrication de la vis et du filin corpusculaire. Il faut en effet que la longueur du filin entre chaque corpuscule soit très précisément ajustée de telle sorte que chaque corpuscule puisse se loger entre les filets tout en maintenant une tension adéquate et identique de corpuscule à corpuscule. Il faut donc une correspondance très précise entre les dimensions de la vis et les dimensions du filin corpusculaire. Cette configuration requiert par ailleurs que la partie la plus saillante du filet de la vis soit suffisamment émoussée pour éviter tout risque de cisaillement du filin par le filet de la vis.

Les deux mécanismes peuvent se combiner si les corpuscules ont une forme ovalaire dont la dimension selon l'axe longitudinal permet l'enclavement entre deux filets et la dimension selon l'axe transversal, perpendiculaire à l'axe du filin, plus grand que la largeur du filet, provoquant son encastrement dans le tissu osseux.

Rappelons ici une caractéristique essentielle de la vis dont la partie distale doit obligatoirement être effilée et conique de manière à créer entre l'âme de la vis et la paroi du tunnel osseux, un espace suffisamment large pour permettre l'incarcération du corpuscule. On comprend aisément qu'une vis trop massive entraînerait inévitablement le refoulement du corpuscule hors du tunnel au lieu de son incarcération dans la paroi du tunnel. Le même effet indésirable se produirait si la vis venait à la rencontre de corpuscules trop volumineux qui seraient alors refoulés et expulsés par la vis, entraînant la faillite du sysytème. Pour que l'incarcération se produise, il faut nécessairement que l'espace situé entre la pointe de la vis et la paroi du tunnel soit plus grand, ou au moins égal au diamètre transversal du corpuscule. Ainsi, la vis pourra progresser sans refouler le corpuscule hors du tunnel, mais au contraire elle l'emprisonnera dans l'espace situé entre elle-même (son âme) et la paroi du tunnel osseux. Une fois que le segment effilé de la vis aura dépassé le corpuscule en l'impactant dans la paroi asseuse, tout accroissement du diamètre de la vis dans son segment proximal ne fera qu'accroître la profondeur d'incarcération du corpuscule.

L'invention sera davantage comprise à la lecture de la description qui suit en tenant compte des dessins annexés, fournis uniquement à titre d'exemples non limitatifs. Les différents éléments structurels de l'invention et leurs agencements sont illustrés dans ces dessins dans lesquels :

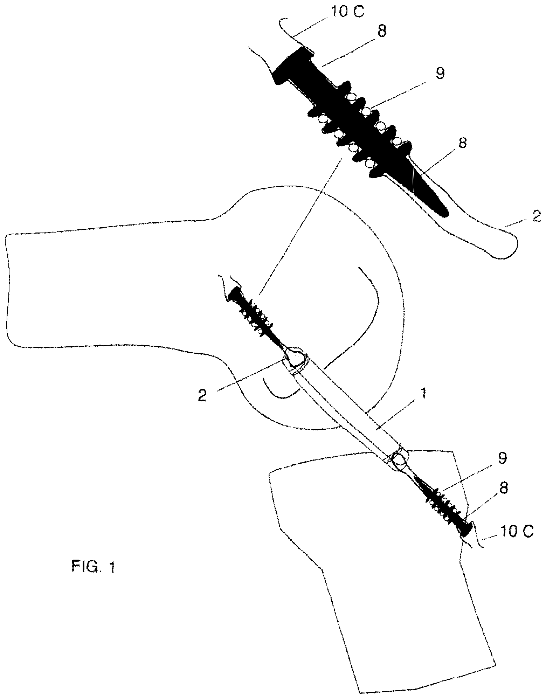

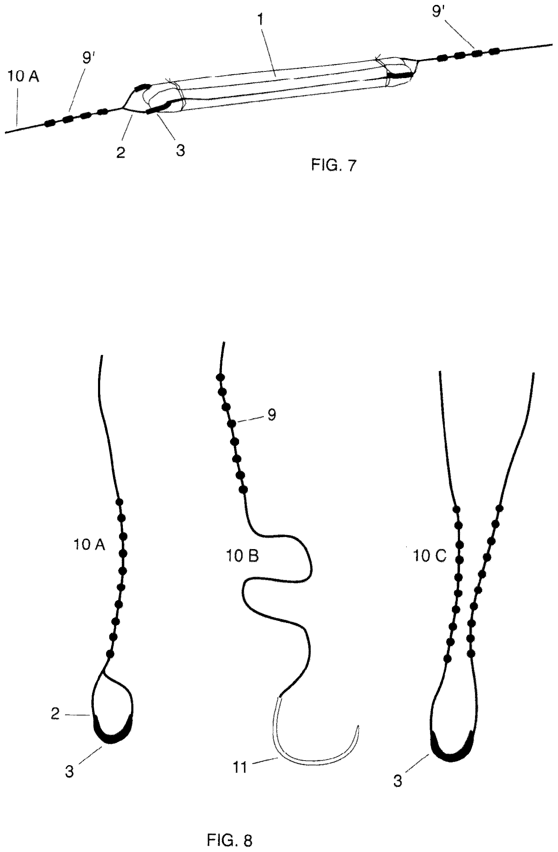

La fig. 1 représente schématiquement l'aspect final d'une greffe avec vis 8, filin 10C (cfr fig 8) et corpuscules 9 de forme sphérique et de diamètre inférieur à la distance entre deux filets de vis, en position sur l'ensemble fémur-tibia, avec, en cartouche, un agrandissement de la vis 8 .

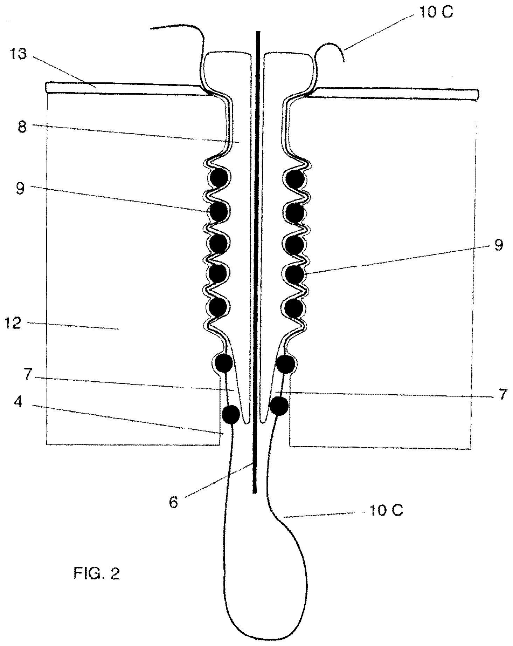

La fig. 2 représente en coupe longitudinale agrandie un segment osseux composé de sa partie spongieuse 12 et de sa couche corticale 13, traversé par un tunnel osseux 4 dans lequel ont été introduits une broche guide 6 ainsi que le filin de suspension 10C muni de ses corpuscules 9. La vis 8, perforée en son centre selon son axe longitudinal , a été enfilée sur la broche guide 6 et pénètre ensuite dans le tunnel osseux 4 selon le trajet imposé par la broche, en écrasant au sein du tissu osseux spongieux 12 chaque corpuscule 9 qui se retrouve ainsi incarcéré au dessus et en dessous par deux filets de vis adjacents et latéralement par l'âme de la vis d'une part et le tissu osseux d'autre part. Notons que l'espace 7 situé entre la portion distale effilée de la vis et la paroi du tunnel permet aux corpuscules de s'y insinuer sans être refoulés par le corps de la vis.

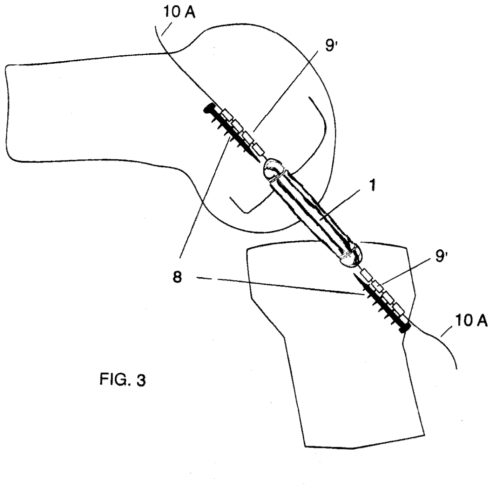

La fig. 3 représente schématiquement l'aspect final d'une greffe 1 avec vis 8, fixant le filin 10A muni de corpuscules oblongs 9' en position sur l'ensemble fémur-tibia,

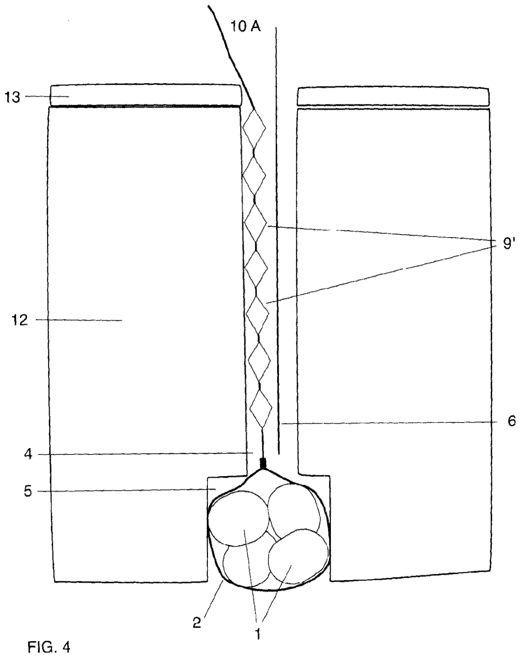

La fig. 4 représente en coupe longitudinale agrandie, un segment d'os spongieux 12 et cortical 13 dans lequel ont été réalisés un tunnel osseux 4 et une logette osseuse 5. Le filin 10A, muni de corpuscules oblongs de forme losangique 9' et d'une anse 2 suspendant la greffe à 4 brins 1 (à 4 brins ?) passe librement dans le tunnel, de dedans en dehors (en direction de son orifice cortical ) et, par traction, entraine à sa suite la greffe 1 jusqu'à ce qu'elle vienne buter sur le fond de la logette osseuse 5. La broche guide 6 passe également librement dans le tunnel osseux 4.

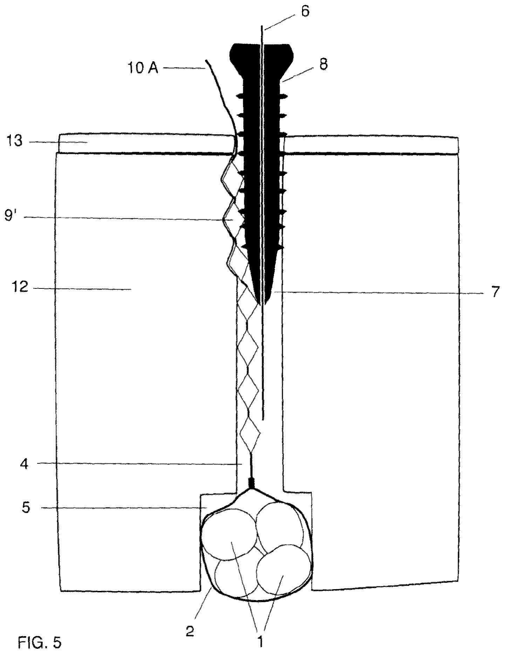

La fig. 5 montre les mêmes composants et illustre le début d'introduction de la vis 8 qui chemine dans le tunnel osseux 4 selon le trajet imposé par la broche guide 6. Notons que l'espace 7 résultant de l'effilement du segment distal de la vis permet à celle ci, lors de sa progression dans le tunnel, de « dépasser » le corpuscule sans le refouler hors du tunnel, l'enfermant ainsi dans l'espace circonscrit par le filet sus et sous-jacent, l'âme de la vis et l'os avoisinant.

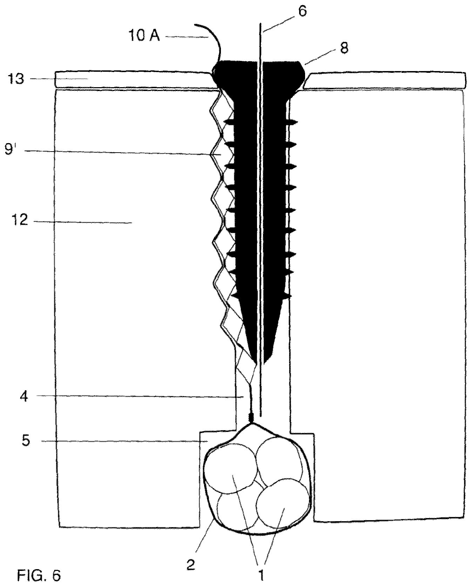

La fig. 6 montre les mêmes composants et illustre la position des corpuscules 9', encastrés dans l'os spongieux 12, après introduction complète de la vis 8.
La fig. 7 représente une greffe 1 suspendue à chacune de ses extrémités par l'anse 2 garnie d'une gaine anti-cisaillement 3, d'un filin 10A muni de corpuscules oblongs 9'.
La fig. 8 représente 3 modes de réalisation de filins corpusculaires qui sont illustrés ici à titre purement exemplatif, n'excluant donc nullement d'autres modes de réalisation éventuellement adaptés à d'autres applications. Le filin 10A de suspension se compose d'un seul filament se terminant à une de ses extrémités par une anse 2 fermée destinée à suspendre la greffe et éventuellement garnie d'une gaine anti-cisaillement 3. Le filin de suspension 10B se termine par une aiguille sertie 11 permettant d'effectuer un laçage de l'extrémité de tissus mous à refixer sur l'os. La filin 10C est replié sur lui-même pour former une anse naturelle destinée à suspendre la greffe, chaque brin de suspension pouvant être garni de corpuscules.

La technique de reconstruction du ligament croisé antérieur au moyen de l'invention est décrite ci-dessous à titre purement exemplatif et ne constitue donc nullement une application exclusive du dispositif et procédé de l'invention.

### Préparation de la greffe.

Le semi-tendineux est prélevé classiquement au moyen d'un stripper.

Comme dans la technique TLS, le tendon est préparé en l'enroulant sur lui-même pour former une greffe courte fermée 1 à 4 brins permettant de la suspendre au moyen d'une anse 2 de traction mise en place à chaque extrémité de la greffe. Dans la technique TLS, les rubans passent librement à travers chaque extrémité de la greffe et peuvent donc être introduits après confection de la greffe.

En cas d'utilisation d'un filin de type 10A, les anses 2 sont fermées et la greffe doit donc être confectionnée en passant les brins tendineux à travers les deux anses déjà mises en place sur la table de préparation.

Le diamètre du filin étant très faible (1 à 2 mm), on peut ajouter à l'anse du filin (soit par fabrication, soit au moment de l'utilisation), une gaine protectrice 3 en matériau textile ou composite, de manière à éviter tout risque de cisaillement de la greffe par le filin. Pour le reste, comme dans la technique TLS, les brins tendineux sont fixés entre eux par un à deux points de suture placés à chaque extrémité de la greffe .

### Préparation des tunnels et logettes osseuses .

Les tunnels et logettes se préparent de façon classique selon des techniques connues et laissées au choix du chirurgien.

Toutes ces techniques impliquent toujours le placement de broches guides 6 sur lesquelles coulissent des mèches ou des fraises perforées servant à percer les tunnels osseux 4 et les logettes 5 d'insertion de la greffe 1.

Au fémur, le creusement des tunnels 4 et logettes 5 se fait, au choix du chirurgien, soit de dedans en dehors à l'aide de viseurs in-out ou encore à l'aide de broches et mèches flexibles (qui laissent au chirurgien le choix du site d'insertion qui lui paraît optimal), soit de dehors en dedans au moyen de viseurs out-in. Au tibia, le placement de la broche guide 6 se fait de dehors en dedans en s'aidant des viseurs adéquats et la logette sera réalisée de façon rétrograde au moyen des outils spécifiques disponibles sur le marché.

Les techniques de perçage des tunnels 4 et logettes 5 mentionnées ci-dessus ne constituent donc pas des caractéristiques spécifiques à la présente invention sauf en ce qui concerne le diamètre des tunnels osseux à réaliser.

Rappelons ici que les techniques classiques de ligamentoplastie imposent, au tibia, le creusement d'un tunnel osseux de diamètre égal voire légèrement supérieur au calibre de la greffe elle-même (de 8 à 11 mm environ). La technique TLS permettait déjà de réduire le calibre des tunnels à un diamètre de 4,5 mm, nécessaire au passage des rubans. La présente technique autorise le creusement d'un tunnel osseux réduit à un diamètre final de 2 à 3 mm et supprime la nécessité de réaliser une logette spécifique pour accueillir l'organe de fixation telle que l'imposait la technique TLS ce qui procure un gain d'économie de tissu osseux non négligeable. En raison de son très faible encombrement, la fixation de type «endobouton », largement utilisée en chirurgie ligamentaire, est, elle aussi, capable de cheminer à travers un tunnel osseux de petit calibre. Hormis la présente invention, C'est donc la seule qui aujourd'hui permet de fixer par suspension une greffe ligamentaire à travers un tunnel osseux de petit calibre, aussi bien au fémur qu'au tibia. Cependant, la fixation obtenue par ce système s'effectue au niveau de la corticale fémorale et tibiale, et donc à distance importante du site d'insertion de la greffe. Or il est aujourd'hui clairement démontré que l'élasticité inévitable des relais textiles disposés entre la greffe et l'organe de fixation corticale constitue un inconvénient mécanique considérable, préjudiciable à la qualité de la reconstruction ligamentaire. La présente invention est donc, à notre connaissance, le seul système qui possède simultanément les trois propriétés fonctionnelles suivantes :
- fixer par suspension une greffe ligamentaire à travers des tunnels osseux de calibre très réduit (2 à 3 mm) à la fois au tibia et au fémur.
- neutraliser l'élasticité des relais de fixation jusqu'au contact de la greffe (ce que faisait déjà TLS)
- supprimer la nécessité de creuser un logement spécifique pour accueillir l'organe de fixation (ce qu'exigeait le système TLS).

### Introduction de la greffe

Avant d'introduire la greffe 1 dans le genou puis dans les logettes osseuses 5, on aura pris le soin de mettre en place dans chacun des tunnels 4, fémoral et tibial, une fine broche guide 6 faisant issue à la peau de la cuisse et du tibia, destinée à guider ultérieurement le trajet de la vis de verrouillage.

Comme dans la technique TLS, la greffe est introduite dans le genou par la voie d'abord antéro-interne. Le filin 10 de suspension du segment fémoral de la greffe est passé dans le tunnel fémoral 2 par son orifice intra-articulaire et est récupéré à la face externe du fémur par un orifice cutané de 2 ou 3 mm, par lequel fait également issue la broche guide fémorale. La traction sur ce filin 10 provoque la pénétration de la greffe 1 dans la logette fémorale 5 jusqu'à ce qu'elle vienne buter sur le fond de cette logette. Une manoeuvre similaire est réalisée au tibia pour mettre en place le segment d'insertion tibial de la greffe 1.

### Verrouillage de la greffe

En maintenant la traction sur le filin fémoral, on introduit sur la broche guide 6 une vis perforée 8 classique utilisée de routine en orthopédie, dont la longueur est choisie en fonction de la longueur du segment de filin à verrouiller.

Il y a lieu de remarquer que cette manoeuvre s'effectue très aisément, sans devoir aborder chirurgicalement la surface externe de l'os concerné et donc sans devoir créer une voie d'abord cutanée (si ce n'est les 2 ou 3 mm nécessaires au passage du filin 10 et de la broche guide 6).

Il y a lieu de noter aussi que la présente invention permet d'éviter les risques de défauts de vissage propres au système TLS (excès ou insuffisance d'introduction de la vis ou encore divergence de la vis par rapport à l'axe du tunnel osseux). En effet, la vis perforée 8 poursuit un trajet nécessairement parallèle au filin 10 grâce à la broche guide 6 écartant tout risque de divergence. D'autre part, grâce à la tête de vis qui s'arrête automatiquement au niveau cortical, le risque d'excès ou d'insuffisance de vissage est également entièrement écarté.

La même manoeuvre permet d'obtenir le verrouillage de la greffe au tibia.

Bien que la présente invention décrive un système particulièrement bien adapté à la reconstruction chirurgicale du ligament croisé antérieur par greffe courte, il ne s'agit nullement d'un choix excluant de ce fait la possibilité d'appliquer le système à d'autres situations rencontrées en chirurgie. Le même système s'applique très bien par exemple à la reconstruction du ligament croisé postérieur, des ligaments latéraux interne et externe mais pourrait également convenir à toute autre procédure nécessitant la fixation de structures molles, tendineuses ou ligamentaires, aux structures osseuses.

D'une manière plus générale, la présente invention divulgue un moyen simple de fixer solidement, au moyen d'une vis à os, perforée ou non, toute espèce de fil ou filin nécessitant d'être fixé dans une structure osseuse, pouvant ainsi éviter de devoir recourir aux dispositifs sophistiqués actuels (ancres intra-osseuses etc..) le plus souvent d'utilisation très coûteuse.

Une autre application potentielle d'un filin simple muni de corpuscules à ses deux extrémités mérite également d'être mentionnée. Il s'agirait de procurer à la greffe un système de protection contre les excès de tension pendant toute sa phase de cicatrisation. Une greffe ligamentaire reste en effet fragile pendant plusieurs mois après sa mise en place et est susceptible de se détendre voire de se rompre si elle est soumise trop tôt à des efforts de traction trop importants.

Lors de la mise en place de la greffe, on pourrait disposer parallèlement à celle-ci, un simple filin muni de corpuscules à chacune de ses extrémités, appelé filin « protecteur », en lui donnant une longueur légèrement plus grande que celle du complexe « filin de suspension-greffe-filin de suspension ». Le filin suspenseur et le filin protecteur seraient fixés de part et d'autre de la greffe dans les mêmes tunnels osseux au moyen des mêmes vis, tibiale et fémorale. Lors d'un mouvement quelconque du genou provoquant un déplacement antérieur du tibia par rapport au fémur, la tension survient d'abord au sein de la greffe elle-même, celle-ci étant plus courte mais, en cas de déplacement plus important, la tension se propage ensuite au filament protecteur, ce qui automatiquement freine voire arrête le mouvement à risque en protégeant la greffe contre un excès de tension voire un risque de rupture.

## Revendications

1. Dispositif de fixation de tissu mou à un tissu osseux, le dispositif comprenant :
- un filin souple (10) formant à l'une de ses extrémités une anse fermée pour recevoir ledit tissu mou et muni de plusieurs corpuscules (9, 9') fixés sur sa longueur, le filin apte à être introduit dans un tunnel osseux définissant une paroi,
- une vis canulée (8) apte à coopérer avec lesdits corpuscules du filin (10) lors du vissage dans ledit tunnel osseux (4) en bloquant ainsi le filin entre un filetage de la vis et le tissu osseux, et
- une broche de guidage (6) apte à coopérer avec la vis (8).

2. Dispositif selon la revendication 1 dans lequell'anse fermée , comprend deux branches munies desdits corpuscules.

3. Dispositif selon la revendication 1 dans lequel le filin se termine par une aiguille sertie (11) permettant d'effectuer un laçage d'une extrémité d'un ligament à refixer sur l'os.

4. Dispositif selon n'importe laquelle des revendications précédentes dans lequel la vis présente un segment effilé distal apte à créer un espace (7) suffisant pour permettre l'insinuation d'un corpuscule entre elle-même et la paroi du tunnel.

5. Dispositif selon n'importe laquelle des revendications précédentes dans lequel la vis présente un calibre croissant au fur et à mesure qu'on se rapproche d'unetête de la vis.

6. Dispositif selon n'importe laquelle une des revendications 1 à 4 dans lequel la vis comprend une section de calibre constant mais de dimension telle qu'elle refoule latéralement le corpuscule au minimum pour entrer en contact avec la paroi du tunnel osseux.

7. Dispositif selon n'importe laquelle des revendications précédentes dans lequel les corpuscules (9) sont, de dimension inférieure ou égale à une distance entre deux segments adjacents de filets de vis, ou oblongs (9'), de dimension longitudinale supérieure ladite distance.

8. Dispositif selon n'importe laquelle des revendications 1 à 7 dans lequel les corpuscules (9) sont sphériques, arrondis, ou de forme anguleuse, de dimension longitudinale inférieure au pas de vis de façon à pouvoir être enfermés et coincés entre deux segments adjacents du filet de la vis (8) et de dimension transversale telle qu'ils soient aptes à affleurer la paroi du tunnel osseux ou s'y encastrer.

9. Dispositif selon n'importe laquelle des revendications précédentes dans lequel on prévoit un filin supplémentaire destiné à être mis en place parallèlement à la greffe mais de longueur légèrement supérieure au complexe greffe-fixations et fixé par les mêmes vis et dans le même tunnel osseux que pour la fixation de la greffe.

10. Dispositif selon n'importe laquelle des revendications 1 à 9 dans lequel, séparément ou en combinaison,
les corpuscules sont éloignés l'un de l'autre de 2 à 20 mm une dimension transversale varie de 1 à 3 mm et dimension longitudinale de 2 à 8 mm, un diamètre du filin varie de 0,5 à 3 mm, et un diamètrede la vis varie de 3 à 8 mm.

## Patentansprüche

1. Vorrichtung zur Befestigung von Weichgewebe an einem Knochengewebe, wobei die Vorrichtung Folgendes umfasst:
- einen flexiblen Draht (10), der an einem seiner Enden eine geschlossene Schlaufe zur Aufnahme des Weichgewebes bildet und mit mehreren entlang seiner Länge befestigten Körperchen (9, 9') versehen ist, wobei der Draht dazu ausgelegt ist, in einen Knochentunnel eingeführt zu werden, der eine Wand definiert,
- eine kanülierte Schraube (8), die ausgelegt ist, um mit den Körperchen des Drahts (10) beim Einschrauben in den Knochentunnel (4) zusammenzuwirken, wodurch der Draht zwischen einem Gewinde der Schraube und dem Knochengewebe blockiert wird, und
- einem Führungsstift (6), der ausgelegt ist, um mit der Schraube (8) zusammenzuwirken.

2. Vorrichtung nach Anspruch 1, wobei die geschlossene Schlaufe zwei Zweige umfasst, die mit den Körperchen versehen sind.

3. Vorrichtung nach Anspruch 1, wobei der Draht in einer gecrimpten Nadel (11) endet, die es ermöglicht, eine Bindung eines Endes eines auf den Knochen wieder zu befestigenden Bandes vorzunehmen.

4. Vorrichtung nach einem der vorhergehenden Ansprüche, wobei die Schraube ein distales verjüngtes Segment aufweist, das ausgelegt ist, um einen Raum (7) zu schaffen, der ausreicht, um das Eindringen eines Körperchens zwischen sich und der Tunnelwand zu ermöglichen.

5. Vorrichtung nach einem der vorhergehenden Ansprüche, wobei die Schraube eine Größe aufweist, die zunimmt, während man sich einem Kopf der Schraube nähert.

6. Vorrichtung nach einem der Ansprüche 1 bis 4, wobei die Schraube eine Sektion mit konstanter Größe aufweist, die jedoch so bemessen ist, dass sie das Körperchen mindestens seitlich verdrängt, um mit der Wand des Knochentunnels in Kontakt zu kommen.

7. Vorrichtung nach einem der vorhergehenden Ansprüche, wobei die Abmessung der Körperchen (9) kleiner oder gleich einem Abstand zwischen zwei benachbarten Segmenten von Schraubengewinden ist oder sie länglich (9') mit Längsabmessung größer als dieser Abstand sind.

8. Vorrichtung nach einem der Ansprüche 1 bis 7, wobei die Körperchen (9) kugelförmig, abgerundet oder eckförmig sind und mit Längsabmessung, die kleiner als die Schraubenteilung ist, so dass sie zwischen zwei benachbarten Segmenten des Gewindes der Schraube (8) eingeschlossen und eingeklemmt werden können, und mit Querabmessung, so dass sie in der Lage sind, mit der Wand des Knochentunnels bündig zu sein oder in diese einzupassen.

9. Vorrichtung nach einem der vorhergehenden Ansprüche, wobei ein zusätzlicher Draht vorgesehen ist, der dazu bestimmt ist, parallel zum Transplantat angebracht zu werden, der jedoch etwas länger als der Transplantat-Befestigungs-Komplex ist und mittels der gleichen Schrauben und im gleichen Knochentunnel wie für die Befestigung des Transplantats befestigt ist.

10. Vorrichtung nach einem der Ansprüche 1 bis 9, wobei die Körperchen einzeln oder in Kombination um 2 bis 20 mm voneinander beabstandet sind, eine Querabmessung von 1 bis 3 mm und eine Längsabmessung von 2 bis 8 mm variieren, ein Drahtdurchmesser von 0,5 bis 3 mm variiert und ein Schraubendurchmesser von 3 bis 8 mm variiert.

## Claims

1. A device for the attachment of soft tissue to a bone tissue, the device comprising:
- a flexible wire (10) forming at one of its ends a closed loop for receiving said soft tissue and provided with several corpuscles (9, 9') fixed along its length, the wire being adapted to be introduced into a bone tunnel defining a wall,
- a cannulated screw (8) adapted to cooperate with said corpuscles of the wire (10) while being screwed into said bone tunnel (4), thus blocking the wire between a thread of the screw and the bone tissue, and
- a guide pin (6) adapted to cooperate with the screw (8) .

2. The device according to claim 1, wherein the closed loop comprises two branches provided with said corpuscles.

3. The device according to claim 1, wherein the wire terminates in a crimped needle (11) which allows tying an end of a ligament to be reattached to the bone.

4. The device according to any one of the preceding claims, wherein the screw has a distal tapered segment adapted to create a space (7) sufficient to allow a corpuscle to find a way between the screw and the wall of the tunnel.

5. The device according to any one of the preceding claims, wherein the screw has a gradually increasing gauge towards a head of the screw.

6. The device according to any one of claims 1 to 4, wherein the screw comprises a constant gauge section but of such a dimension that it laterally drives the corpuscle at least to come into contact with the wall of the bone tunnel.

7. The device according to any one of the preceding claims, wherein the corpuscles (9) are smaller than or equal to a distance between two adjacent segments of screw threads, or oblong (9'), having a longitudinal dimension greater than said distance.

8. The device according to any one of claims 1 to 7, wherein the corpuscles (9) are spherical, rounded or angular shaped, having a longitudinal dimension lower than the screw pitch so as to be able to be enclosed and locked between two adjacent segments of the thread of the screw (8) and with a transverse dimension such that they are adapted to be flush with the wall of the bone tunnel or to be embedded therein.

9. The device according to any one of the preceding claims, wherein an extra wire is provided, intended to be arranged parallel to the graft but with a slightly longer length than the graft-attachment assembly and attached by the same screws and in the same bone tunnel as for attaching the graft.

10. The device according to any one of claims 1 to 9 wherein, separately or in combination, the corpuscles are distanced from each other by 2 to 20 mm, a transverse dimension ranges from 1 to 3 mm and a longitudinal dimension from 2 to 8 mm, a diameter of the wire ranges from 0.5 to 3 mm, and a diameter of the screw ranges from 3 to 8 mm.
